# EUROPÄISCHE PATENTSCHRIFT

(11) **EP 0 476 333 B1**
(45) Veröffentlichungstag und Bekanntmachung des Hinweises auf die Patenterteilung: **30.11.1994**
(21) Anmeldenummer: 91113958.2
(22) Anmeldetag: 21.08.1991
(51) Int. Cl.: C07C 53/08, C07C 53/12, C07C 51/12, C07C 51/56

(54) **Verfahren zur gleichzeitigen Herstellung von Essigsäure und Essigsäureanhydrid**
Process for simultaneous production of acetic acid and acetanhydride
Procédé de production simultané d'acide acétique et d'anhydride acétique

(30) Priorität: 21.09.1990 DE 4029917
(43) Veröffentlichungstag der Anmeldung: 25.03.1992
(73) Patentinhaber: HOECHST AKTIENGESELLSCHAFT, 65926 Frankfurt am Main (DE)
(72) Erfinder: Erpenbach, Heinz, Dr., W-5000 Köln (DE); Gradl, Reinhard, Dr., W-5042 Erfstadt (DE); Jägers, Erhard, Dr., W-5303 Bornheim (DE); Seidel, Andreas, Dr., W-5000 Köln (DE)

(56) Entgegenhaltungen:
- DE-A- 2 749 955
- GB-A- 742 740
- US-A- 4 002 678

## Beschreibung

Die Erfindung betrifft ein Verfahren zur gleichzeitigen Herstellung von Essigsäure und Essigsäureanhydrid durch Umsetzung von Gemischen aus Methanol und Methylacetat sowie ggf. Dimethylether mit Kohlenmonoxid.

Essigsäure und Essigsäureanhydrid sind bedeutende aliphatische Zwischenprodukte. Die überwiegende Menge wird zur Herstellung von Vinylacetat bzw. Cellulose-Acetat verwendet.

Ein Verfahren zur gleichzeitigen Herstellung von Essigsäure und Essigsäureanhydrid ist aus der DE-A-38 23 645 bekannt. Dort wird die Umsetzung in Gegenwart eines Katalysatorsystems, das Carbonylkomplexe von Edelmetallen der Gruppe VIII des Periodensystems der Elemente, insbesondere von Rhodium enthält, durchgeführt. Die geringe Verfügbarkeit des Rhodiums hat ein enorm hohes Kostenniveau zur Folge, wodurch das Verfahren nachteilig beeinflußt wird. Darüber hinaus verlangt der hohe Preis eine Rückgewinnung des Rhodiums aus der verbrauchten Katalysatorlösung, was sich äußerst kostenintensiv gestaltet. Die Suche nach einem Ersatz-Katalysator für das kostspielige Rhodium hat deshalb an vielen Orten eine hohe Priorität.

Die DE-A-26 58 216 beschreibt die Herstellung von Essigsäureanhydrid durch Umsetzung von Methylacetat oder Dimethylether mit Kohlenmonoxid in Gegenwart eines Katalysators aus Nickel und Chrom in Gegenwart eines Promotors, der eine Organostickstoff- oder eine Organophosphor-Verbindung mit 3-wertigem Stickstoff oder Phosphor enthält. Bei dem Verfahren entsteht keine Essigsäure.

Auch in dem Verfahren zur Herstellung von Essigsäureanhydrid gemäß der DE-C-31 51 371, bei dem Nickel zusammen mit Molybdän oder Wolfram als Katalysator eingesetzt wird, wird keine Essigsäure erhalten.

Demgegenüber beschreiben die DE-C-27 49 954 und 27 49 955 Verfahren zur Herstellung von Essigsäure aus Methylacetat in Gegenwart von Nickel als Katalysator. Bei diesen Verfahren wird kein Essigsäureanhydrid gebildet.

Es war die Aufgabe gestellt, sowohl Essigsäure als auch Essigsäureanhydrid im gleichen Reaktionssystem unter wasserfreien Bedingungen herzustellen. Wasserfreie Bedingungen haben den Vorteil einer wesentlich geringeren Korrosivität. Mit dem Verfahren soll die Möglichkeit geschaffen werden, das Mengenverhältnis von Carbonsäure zu Carbonsäureanhydrid den jeweiligen wirtschaftlichen Erfordernissen anpassen zu können. Weiter soll das Verfahren bei mittleren Drucken mit großen Raumzeitausbeuten und hohen Ausbeuten arbeiten.

Die vorliegende Erfindung betrifft somit ein Verfahren zur gleichzeitigen Herstellung von Essigsäure und Essigsäureanhydrid aus einem Ausgangsgemisch von Methanol und Methylacetat sowie gegebenenfalls Dimethylether durch Umsetzung mit Kohlenmonoxid in einer Reaktionszone an einem Katalysatorsystem unter wasserfreien Bedingungen und ist dadurch gekennzeichnet, daß man
a) in Gegenwart eines Katalysatorsystems, bestehend aus einem Nichtedelmetall der Gruppe VIII des Periodensystems, Methyliodid, einer Alkali-, Organophosphonium- oder Organoammonium-Verbindung als Promotor und gegebenenfalls einer Verbindung eines Nichtedelmetalls der Gruppen IV bis VII des Periodensystems als Co-Promotor, arbeitet;
b) ein molares Verhältnis von Methanol zu Methylacetat sowie gegebenenfalls Dimethylether von 10 : 1 bis 1 : 10 als Ausgangsgemisch einsetzt und
c) die Umsetzung unter einem Druck von 25 bis 200 bar und einer Temperatur von 150 bis 250 °C durchführt.

Das erfindungsgemäße Verfahren kann wahlweise noch dadurch ausgestaltet sein, daß man
aa) als Nichtedelmetall der Gruppe VIII des Periodensystems Nickel verwendet;
bb) als Katalysatorkomponente Nickeliodid, Nickelchlorid, Nickelcarbonyl, Nickelacetylacetonat oder Nickelacetat verwendet;
cc) als Promotor eine Alkali-, eine Organophosphonium- oder Organoammonium-Verbindung in Form ihres Acetats oder Iodids verwendet;
dd) als Alkalisalz Lithiumsalz verwendet;
ee) als Organophosphoniumverbindung Methyltributyl-, Methyltriphenyl-, Tetrabutyl- oder Dimethyldibutylphosphoniumiodid verwendet;
ff) als Organoammoniumverbindung N,N-Dimethylimidazolium-, N-Methylpyridinium-, N-Methyl-3-picolinium- oder N-Methylchinoliniumiodid verwendet;
gg) die Konzentration des Nichtedelmetalles der Gruppe VIII des Periodensystems in der Reaktionsmischung zwischen 0,01 und 0,75, insbesondere 0,05 und 0,5 mol/l, einstellt;
hh) die Konzentration des Methyliodids in der Reaktionsmischung zwischen 0,1 und 7,5, insbesondere 0,5 und 5 mol/l, einstellt;
ii) die Konzentration der als Promotor eingesetzten Alkali-, Organophosphonium- oder Organoammonium-Verbindung in der Reaktionsmischung zwischen 0,05 und 4,5, insbesondere 0,25 und 3,0 mol/l, einstellt;
jj) als Co-Promotor Verbindungen von Ti, Zr, V, Nb, Ta, Cr, Mo, W, Mn oder Re verwendet;
kk) die Konzentration des Co-Promotors in der Reaktionsmischung zwischen 0,01 und 0,5, insbesondere 0,05 und 0,3 mol/l, einstellt;
ll) dem eingesetzten Kohlenmonoxid bis zu 15 Vol.-% Wasserstoff zusetzt;
mm) die Umsetzung unter einem Druck von 50 bis 100 bar und einer Temperatur von 175 bis 225 °C durchführt;
nn) das Carbonylierungsverfahren sowohl kontinuierlich als auch diskontinuierlich betreibt.

Ein besonderer Vorteil des erfindungsgemäßen Verfahrens liegt darin, daß sich durch Variation des Verhältnisses des Ausgangsgemisches von Methanol zu Methylacetat sowie gegebenenfalls Dimethylether praktisch jedes Mengenverhältnis von Carbonsäure zu Carbonsäureanhydrid einstellen läßt, so daß das Verfahren schnell wechselnden Erfordernissen angepaßt werden kann. Das zur Reaktion eingesetzte Kohlenmonoxid muß nicht unbedingt rein sein. Kleinere Mengen an Inertgasen wie Kohlendioxid, Stickstoff oder Methan stören die Carbonylierungsreaktion nicht, wenn ein genügend hoher Kohlenmonoxidpartialdruck im Reaktor aufrechterhalten wird.

Wasserstoffgehalte bis zu 15 Vol.-% wirken sich positiv auf die Katalysatoraktivität aus, verringern jedoch die Selektivität des Verfahrens durch Bildung von Hydrierungsprodukten wie z.B. Ethylidendiacetat.

Als Katalysator kann jedes Nichtedelmetall der Gruppe VIII des Periodensystems eingesetzt werden. Die höchste Aktivität besitzt das Nickel. Alle Nickelsalze, die unter den Reaktionsbedingungen löslich sind und den aktiven Carbonylkomplex bilden, können eingesetzt werden.

Von den als Promotor einsetzbaren Alkalisalzen sind Salze des Lithiums wirksamer als die des Natriums oder Kaliums.

Die gegebenenfalls als Co-Promotor einsetzbaren Nichtedelmetalle der Gruppen IV, V, VI und VII des Periodensystems werden in löslicher Form, insbesondere als Acetate, Acetylacetonate oder Carbonyle, eingesetzt.

### Beispiel 1

In einem 1-l-Rührautoklaven aus Edelstahl (Hastelloy B2) wurden 3 mol Methylacetat, 3 mol Methanol, 1 mol Methyliodid, 0,1 mol Nickeliodid und 0,4 mol Methyltributylphosphoniumiodid vorgelegt. Anschließend wurde durch Aufdrücken von Kohlenmonoxid ein Druck von 25 bar eingestellt. Nach dem Aufheizen auf die Reaktionstemperatur von 195 °C wurde durch kontinuierliches Nachdrücken von Kohlenmonoxid 80 Minuten lang ein Gesamtdruck von 80 bar aufrechterhalten. Nach dem Abkühlen und Entspannen wurden aus 590 ml Reaktionsprodukt 291 g Essigsäureanhydrid (2,85 mol) und 176 g Essigsäure (2,94 mol) isoliert. Diese Werte entsprechen Ausbeuten von 95 %, bezogen auf das eingesetzte Methylacetat, und von 98 %, bezogen auf das eingesetzte Methanol. Die Raumzeitausbeute, bezogen auf das flüssige Reaktionsvolumen, beträgt 594 g Essigsäure + Essigsäureanhydrid/l . h.

### Beispiel 2

In dem 1-l-Rührautoklaven wurden 1 mol Methylacetat, 5 mol Methanol, 2 mol Methyliodid, 0,1 mol Nickeliodid und 0,6 mol Methyltributylphosphoniumiodid vorgelegt. Durch Aufdrücken eines Gemisches aus 95 Vol.-% Kohlenmonoxid und 5 Vol.-% Wasserstoff wurde ein Druck von 25 bar eingestellt. Nach dem Aufheizen auf die Reaktionstemperatur von 195 °C wurde durch kontinuierliches Nachdrücken derselben Gasmischung 51 Minuten lang ein Gesamtdruck von 80 bar aufrechterhalten. Nach dem Abkühlen und Entspannen wurden aus 640 ml Reaktionsprodukt 97 g Essigsäureanhydrid (0,95 mol) und 297 g Essigsäure (4,95 mol) isoliert. Hieraus errechnen sich Ausbeuten von 95 %, bezogen auf das eingesetzte Methylacetat, und von 99 %, bezogen auf das eingesetzte Methanol. Die Raumzeitausbeute, bezogen auf das flüssige Reaktionsvolumen, beträgt 724 g Essigsäure + Essigsäureanhydrid/l . h.

### Beispiel 3

In dem 1-l-Rührautoklaven wurden 5 mal Methylacetat, 1 mol Methanol, 1 mol Methyliodid, 1 mol Essigsäure, 0,1 mol Nickeliodid und 0,4 mol Methyltributylphasphoniumiodid vorgelegt. Durch Aufdrücken eines Gemisches aus 92 Vol.-% Kohlenmonoxid und 8 Vol.-% Wasserstoff wurde ein Druck von 25 bar eingestellt. Nach dem Aufheizen auf die Reaktionstemperatur von 195 °C wurde durch kontinuierliches Nachdrücken derselben Gasmischung 83 Minuten lang ein Gesamtdruck von 100 bar aufrechterhalten. Nach dem Abkühlen und Entspannen wurden aus 734 ml Reaktionsprodukt 479 g Essigsäureanhydrid (4,7 mol) und 118 g Essigsäure isoliert. Nach Abzug der vorgelegten Essigsäuremenge verbleiben 58 g Essigsäure (0,97 mal). Hieraus errechnen sich Ausbeuten von 94 %, bezogen auf das eingesetzte Methylacetat, und von 97 %, bezogen auf das eingesetzte Methanol. Die Raumzeitausbeute, bezogen auf das flüssige Reaktionsvolumen, beträgt 529 g Essigsäure + Essigsäureanhydrid/l . h.

### Beispiel 4

Das Beispiel 4 entspricht dem Beispiel 1 mit dem Unterschied, daß anstelle des Methyltributylphosphoniumiodids 0,2 mol N,N-Dimethylimidazoliumiodid eingesetzt wurden. Aus 590 ml Reaktionsprodukt wurden 294 g Essigsäureanhydrid (2,88 mol) und 178 g Essigsäure (2,97 mol) isoliert. Hieraus errechnen sich Ausbeuten von 96 %, bezogen auf das eingesetzte Methylacetat, und von 99 %, bezogen auf das eingesetzte Methanol. Die Raumzeitausbeute, bezogen auf das flüssige Reaktionsvolumen, beträgt 600 g Essigsäure + Essigsäureanhydrid/l . h.

### Beispiel 5

In dem 1-l-Rührautoklaven wurden 3 mol Methylacetat, 3 mol Methanol, 1 mol Methyliodid, 0,05 mol Nickeliodid und 0,3 mol Lithiumacetat vorgelegt. Durch Aufdrücken eines Gemisches aus 92 Vol.-% Kohlenmonoxid und 8 Vol.-% Wasserstoff wurde ein Druck von 30 bar eingestellt. Nach dem Aufheizen auf die Reaktionstemperatur von 195 °C wurde durch kontinuierliches Nachdrücken derselben Gasmischung 90 Minuten lang ein Gesamtdruck von 100 bar aufrechterhalten. Nach dem Abkühlen und Entspannen wurden aus 580 ml Reaktionsprodukt 291 g Essigsäureanhydrid (2,85 mol) und 178 g Essigsäure (2,97 mol) isoliert. Hieraus errechnen sich Ausbeuten von 95 %, bezogen auf das eingesetzte Methylacetat, und von 99 %, bezogen auf das eingesetzte Methanol. Die Raumzeitausbeute, bezogen auf das flüssige Reaktionsvolumen, beträgt 539 g Essigsäure + Essigsäureanhydrid/l . h.

### Beispiel 6

In dem 1-l-Rührautoklaven wurden 3 mol Methylacetat, 0,5 mol Methanol, 2 mol Methyliodid, 1 mol Essigsäure, 0,2 mol Nickelacetat, 0,4 mol Methyltributylphosphoniumiodid und 0,05 mol Zirkoniumacetylacetonat vorgelegt. Durch Aufdrücken eines Gemisches aus 95 Vol.-% Kohlenmonoxid und 5 Vol.-% Wasserstoff wurde ein Druck von 25 bar eingestellt. Nach dem Aufheizen auf 190 °C Reaktionstemperatur wurde über einen Zeitraum von 45 Minuten durch kontinuierliches Nachdrücken derselben Gasmischung ein Gesamtdruck von 100 bar aufrechterhalten. Aus 609 ml Reaktionsprodukt wurden 297 g Essigsäureanhydrid (2,91 mol) und 90 g Essigsäure isoliert. Nach Abzug der eingesetzten Essigsäuremenge verbleiben 30 g Essigsäure (0,495 mol). Hieraus errechnen sich Ausbeuten von 97 %, bezogen auf das eingesetzte Methylacetat, und 99 %, bezogen auf das eingesetzte Methanol. Die Raumzeitausbeute, bezogen auf das flüssige Reaktionsvolumen, beträgt 716 g Essigsäure + Essigsäureanhydrid/l . h.

### Beispiel 7

In dem 1-l-Rührautoklaven wurden 0,5 mol Methylacetat, 3,5 mol Methanol, 2 mol Methyliodid, 0,1 mol Nickelcarbonyl, 0,6 mol Methyltributylphosphoniumiodid und 0,5 mol Vanadiumhexacarbonyl vorgelegt. Durch Aufdrücken eines Gemisches aus 92 Vol.-% Kohlenmonoxid und 8 Vol.-% Wasserstoff wurde ein Druck von 25 bar eingestellt. Nach dem Aufheizen auf 205 °C Reaktionstemperatur wurde über einen Zeitraum von 30 Minuten durch kontinuierliches Nachdrücken derselben Gasmischung ein Gesamtdruck von 80 bar aufrechterhalten. Aus 532 ml Reaktionsprodukt wurden 49 g Essigsäureanhydrid (0,475 mol) und 208 g Essigsäure (3,465 mol) isoliert. Hieraus errechnen sich Ausbeuten von 95 %, bezogen auf das eingesetzte Methylacetat, und 99 %, bezogen auf das eingesetzte Methanol. Die Raumzeitausbeute, bezogen auf das flüssige Reaktionsvolumen, beträgt 966 g Essigsäure + Essigsäureanhydrid/l . h.

### Beispiel 8

Das Beispiel 7 wurde wiederholt mit dem Unterschied, daß anstelle von Vanadiumhexacarbonyl 0,05 mol Chromacetylacetonat vorgelegt wurden. Aus 535 ml Reaktionsprodukt wurden 48 g Essigsäureanhydrid (0,47 mol) und 205 g Essigsäure (3,43 mol) isoliert. Hieraus errechnen sich Ausbeuten von 94 %, bezogen auf das eingesetzte Methylacetat, und von 98 %, bezogen auf das eingesetzte Methanol. Die Raumzeitausbeute, bezogen auf das flüssige Reaktionsvolumen, beträgt 946 g Essigsäure + Essigsäureanhydrid/l . h.

### Beispiel 9

In dem 1-l-Rührautoklaven wurden 1 mol Methylacetat, 3,5 mol Methanol, 1,5 mol Methyliodid, 1 mol Essigsäure, 0,2 mol Nickelacetat, 0,4 mol N,N-Dimethylimidazoliumiodid und 0,05 mol Dirheniumdecacarbonyl eingesetzt. Durch Aufdrücken eines Gemisches aus 95 Vol.-% Kohlenmonoxio und 5 Vol.-% Wasserstoff wurde ein Druck von 25 bar eingestellt. Nach dem Aufheizen auf die Reaktionstemperatur von 195 °C wurde durch kontinuierliches Nachdrücken derselben Gasmischung 35 Minuten lang ein Gesamtdruck von 100 bar aufrechterhalten. Nach dem Abkühlen und Entspannen wurden aus 535 ml Reaktionsprodukt 98 g Essigsäureanhydrid (0,96 mol) und 268 g Essigsäure isoliert. Nach Abzug der eingesetzten Essigsäuremenge verbleiben 208 g Essigsäure (3,465 mol). Hieraus errechnen sich Ausbeuten von 96 %, bezogen auf Methylacetat, und von 99 %, bezogen auf Methanol. Die Raumzeitausbeute, bezogen auf das flüssige Reaktionsvolumen, beträgt 981 g Essigsäure + Essigsäureanhydrid/l . h.

### Beispiel 10

Im 1-l-Rührautoklaven wurden 2 mol Methylacetat, 2 mol Dimethylether, 2 mol Methanol, 1 mol Methyliodid, 0,15 mol Nickelacetylacetonat und 0,4 mol Methyltributylphosphoniumiodid vorgelegt. Durch Aufdrücken von Kohlenmonoxid wurde ein Druck von 30 bar eingestellt. Nach dem Aufheizen auf eine Reaktionstemperatur von 195 °C wurde durch kontinuierliches Nachdrücken von Kohlenmonoxid 120 Minuten lang ein Gesamtdruck von 100 bar aufrechterhalten. Nach dem Abkühlen und Entspannen wurden aus 590 ml Reaktionsprodukt 387 g Essigsäureanhydrid (3,79 mol) und 117 g Essigsäure (1,95 mol) isoliert. Hieraus errechnen sich Ausbeuten von 94,75 %, bezogen auf die Summe des eingesetzten Methylacetats/Dimethylethers, und 97,5 %, bezogen auf das eingesetzte Methanol. Die Raumzeitausbeute, bezogen auf das flüssige Reaktionsvolumen, beträgt 427 g Essigsäure + Essigsäureanhydrid/l . h.

### Beispiel 11

Im 1-l-Rührautoklaven wurden 3 mol Methylacetat, 3 mol Methanol, 1 mol Methyliodid, 0,2 mol Nickelcarbonyl und 0,6 mol Methyltributylphosphoniumiodid vorgelegt. Durch Aufdrücken von Kohlenmonoxid wurde ein Druck von 30 bar eingestellt. Nach dem Aufheizen auf die Reaktionstemperatur von 195 °C wurde durch kontinuierliches Nachdrücken von Kohlenmonoxid 10 Minuten lang ein Gesamtdruck von 80 bar aufrechterhalten. Nach dem Abkühlen und Entspannen wurden aus 685 ml Reaktionsprodukt 61 g Essigsäureanhydrid (0,6 mol), 177 g Essigsäure (2,95 mol) und 176 g Methylacetat (2,38 mol) isoliert. Diese Werte entsprechen Ausbeuten von 96,8 %, bezogen auf das eingesetzte Methylacetat, und von 98,3 %, bezogen auf das eingesetzte Methanol. Die Raumzeitausbeute, bezogen auf das flüssige Reaktionsvolumen, beträgt 1428 g Essigsäure + Essigsäureanhydrid/l . h.

### Beispiel 12 (Kontinuierliches Verfahren)

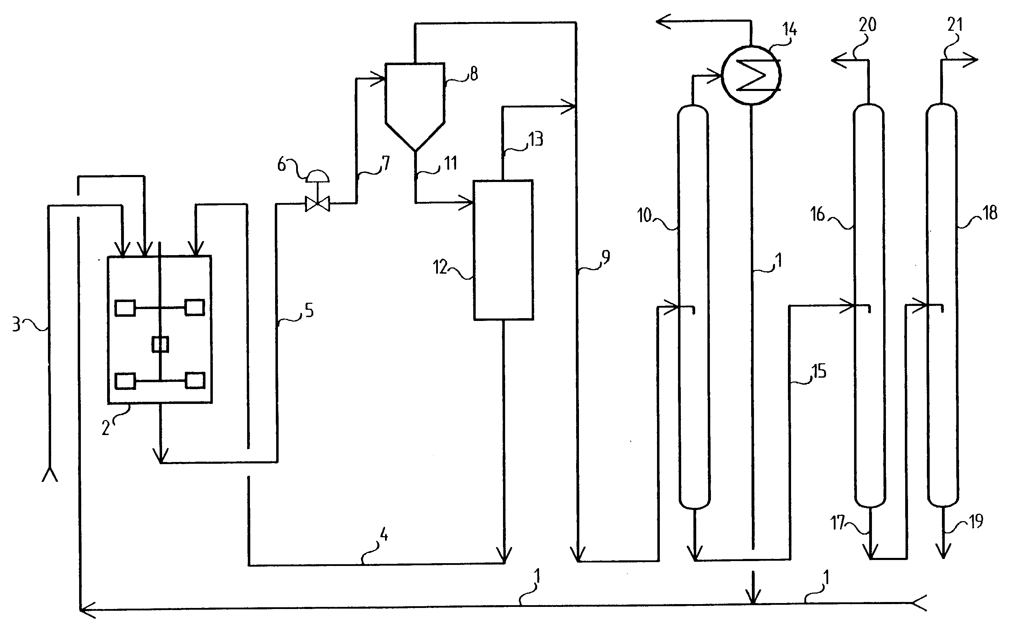

Die Carbonylierung erfolgte bei einer Temperatur von 195 °C unter einem Gesamtdruck von 80 bar. Das genutzte Reaktorvolumen betrug 4,5 l. Das Reaktionsgemisch enthielt den Nickelkomplex, Methyltributylphosphoniumiodid und Methyliodid im Molverhältnis von 1 : 3,5 : 10. Die Konzentrationen an Nichtedelmetall betrugen 0,3 mol Ni/l, an Methyliodid 3 mol/l und an Methyltributylphosphoniumiodid 1,05 mol/l Reaktionsgemisch. Stündlich wurden 1 kg Methylacetat (13,5 mol), 1,85 kg Methanol (57,8 mol) und 11,7 l Leichtsieder (Methyliodid, Methylacetat) über Leitung 1 (vergl. die Figur) dem Reaktor 2 zugeführt. Durch Leitung 3 wurden 2 kg Kohlenmonoxid (71,4 mol) in den Reaktor gedrückt. Über Leitung 4 wurden stündlich 10,0 l Katalysatorlösung in den Reaktor 2 dosiert. Über die Leitung 5 wurden aus dem Reaktor 2 stündlich 26,3 l Reaktionsprodukt entnommen. Hieraus ergibt sich eine mittlere Verweilzeit im Reaktor 2 von etwa 10 Minuten. Unter Konstanthaltung des Volumens der Reaktionsprodukte wurden aus dem Reaktor 2 die Reaktionsprodukte über das Entspannungsventil 6 durch Entspannen auf 1 bar entnommen und gelangten über Leitung 7 in den Abscheider 8, in dem sich eine mittlere Temperatur von 95 °C einstellte. Dampfförmig strömten 14,7 kg/h Reaktionsprodukt aus dem Abscheider 8 über die Leitung 9 in die Leichtsiederkolonne 10; flüssige Reaktionsprodukte aus dem Abscheider 8 flossen über die Leitung 11 in die Trennstufe 12, wo unter Normaldruck und 145 °C weitere 7,60 kg/h Reaktionsprodukte verdampften und durch die Leitungen 13 und 9 der Leichtsiederkolonne 10 zugeführt wurden. Die in der Trennstufe 12 flüssig abgetrennte katalysatorhaltige Lösung wurde über Leitung 4 in den Reaktor 2 rückgeführt.

In der Leichtsiederkolonne 10 erfolgte unter Normaldruck bei 126 °C Sumpf- und 70 °C Kopftemperatur die Abtrennung der Leichtsieder Methyliodid und Methylacetat, die über Leitung 1 in den Reaktor 2 rückgeführt wurden. 0,08 kg/h nichtkondensierbare Gase (CO, CO₂, CH₄, N₂) wurden aus dem Kondensator 14 ausgeschleust.

4,765 kg/h Sumpfprodukt der Leichtsiederkolonne 10 wurden über Leitung 15 entnommen und in der Kolonne 16, die unter einem Druck von 150 mbar bei 70 °C Kopf- und 99 °C Sumpftemperatur betrieben wurde, 3,4 kg/h reine Essigsäure (56,7 mol) durch Leitung 20 abgezogen. Dies entspricht einer Ausbeute von 98 %, bezogen auf das eingesetzte Methanol.

Das Sumpfprodukt der Kolonne 16 wurde über die Leitung 17 entnommen und in der Kolonne 18, die unter einem Druck von 150 mbar betrieben wurde, fraktioniert. 1,29 kg/h reines Essigsäureanhydrid (12,65 mol) wurden bei einer Kopftemperatur von 90 °C und einer Sumpftemperatur von 104 °C über die Leitung 21 erhalten. Dies entspricht einer Ausbeute von 93,7 %, bezogen auf das umgesetzte Methylacetat.

0,075 kg/h Hochsieder wurden als Sumpfprodukt der Kolonne 18 über Leitung 19 entnommen.

Die Ausbeute an Essigsäure und Essigsäureanhydrid entspricht 97,1 %, bezogen auf den CO-Einsatz. Die Raumzeitausbeute, bezogen auf das genutzte Reaktorvolumen, beträgt 1042 g Essigsäure und Essigsäureanhydrid/l . h.

## Patentansprüche

1. Verfahren zur gleichzeitigen Herstellung von Essigsäure und Essigsäureanhydrid aus einem Ausgangsgemisch von Methanol und Methylacetat sowie gegebenenfalls Dimethylether durch Umsetzung mit Kohlenmonoxid in einer Reaktionszone an einem Katalysatorsystem unter wasserfreien Bedingungen, dadurch gekennzeichnet, daß man
a) in Gegenwart eines Katalysatorsystems, bestehend aus einem Nichtedelmetall der Gruppe VIII des Periodensystems, Methyliodid, einer Alkali-, Organophosphonium- oder Organoammonium-Verbindung als Promotor und gegebenenfalls einer Verbindung eines Nichtedelmetalls der Gruppen IV bis VII des Periodensystems als Co-Promotor, arbeitet;
b) ein molares Verhältnis von Methanol zu Methylacetat sowie gegebenenfalls Dimethylether von 10 : 1 bis 1 : 10 als Ausgangsgemisch einsetzt und
c) die Umsetzung unter einem Druck von 25 bis 200 bar und einer Temperatur von 150 bis 250 °C durchführt.

2. Verfahren nach Anspruch 1, dadurch gekennzeichnet, daß man als Nichtedelmetall der Gruppe VIII des Periodensystems Nickel verwendet.

3. Verfahren nach Anspruch 1 oder 2, dadurch gekennzeichnet, daß man als Katalysatorkomponente Nickeliodid, Nickelchlorid, Nickelcarbonyl, Nickelacetylacetonat oder Nickelacetat verwendet.

4. Verfahren nach einem der Ansprüche 1 bis 3, dadurch gekennzeichnet, daß man als Promotor eine Alkali-, eine Organophosphonium- oder Organoammonium-Verbindung in Form ihres Acetats oder Iodids verwendet.

5. Verfahren nach einem der Ansprüche 1 bis 4, dadurch gekennzeichnet, daß man als Alkaliverbindung Lithiumsalz verwendet.

6. Verfahren nach einem der Ansprüche 1 bis 4, dadurch gekennzeichnet, daß man als Organophosphoniumverbindung Methyltributyl-, Methyltriphenyl-, Tetrabutyl- oder Dimethyldibutylphosphoniumiodid verwendet.

7. Verfahren nach einem der Ansprüche 1 bis 4, dadurch gekennzeichnet, daß man als Organoammoniumverbindung N,N-Dimethylimidazolium-, N-Methylpyridinium-, N-Methyl-3-picolinium- oder N-Methylchinoliniumiodid verwendet.

8. Verfahren nach einem der Ansprüche 1 bis 7, dadurch gekennzeichnet, daß man die Konzentration des Nichtedelmetalles der Gruppe VIII des Periodensystems in der Reaktionsmischung zwischen 0,01 und 0,75, insbesondere 0,05 und 0,5 mol/l, einstellt.

9. Verfahren nach einem der Ansprüche 1 bis 8, dadurch gekennzeichnet, daß man die Konzentration des Methyliodids in der Reaktionsmischung zwischen 0,1 und 7,5, insbesondere 0,5 und 5 mol/l, einstellt.

10. Verfahren nach einem der Ansprüche 1 bis 9, dadurch gekennzeichnet, daß man die Konzentration der als Promotor eingesetzten Alkali-, Organophosphonium- oder Organoammonium-Verbindung in der Reaktionsmischung zwischen 0,05 und 4,5, insbesondere 0,25 und 3,0 mol/l, einstellt.

11. Verfahren nach einem der Ansprüche 1 bis 10, dadurch gekennzeichnet, daß man als Co-Promotor Verbindungen von Ti, Zr, V, Nb, Ta, Cr, Mo, W, Mn oder Re verwendet.

12. Verfahren nach einem der Ansprüche 1 bis 11, dadurch gekennzeichnet, daß man die Konzentration des Co-Promotors in der Reaktionsmischung zwischen 0,01 und 0,5, insbesondere 0,05 und 0,3 mol/l, einstellt.

13. Verfahren nach einem der Ansprüche 1 bis 12, dadurch gekennzeichnet, daß man dem eingesetzten Kohlenmonoxid bis zu 15 Vol.-% Wasserstoff zusetzt.

14. Verfahren nach einem der Ansprüche 1 bis 13, dadurch gekennzeichnet, daß man die Umsetzung unter einem Druck von 50 bis 100 bar und einer Temperatur von 175 bis 225 °C durchführt.

15. Verfahren nach einem der Ansprüche 1 bis 14, dadurch gekennzeichnet, daß man das Carbonylierungsverfahren sowohl kontinuierlich als auch diskontinuierlich betreibt.

## Claims

1. A process for the simultaneous preparation of acetic acid and acetic anhydride by reacting a starting mixture of methanol and methyl acetate and, if desired, dimethyl ether with carbon monoxide in a reaction zone on a catalyst system under anhydrous conditions, which comprises
a) working in the presence of a catalyst system comprising a non-noble metal from Group VIII of the Periodic Table, methyl iodide, an alkali metal compound, organophosphonium compound or organoammonium compound as promoter and, if desired, a compound of a non-noble metal from Groups IV to VII of the Periodic Table as copromoter;
b) employing a molar ratio between methanol and methyl acetate and, if used, dimethyl ether of from 10 : 1 to 1 : 10 as the starting mixture, and
c) carrying out the reaction under a pressure of from 25 to 200 bar and at a temperature of from 150 to 250°C.

2. The process as claimed in claim 1, wherein the non-noble metal from Group VIII of the Periodic Table is nickel.

3. The process as claimed in claim 1 or 2, wherein the catalyst component used is nickel iodide, nickel chloride, nickel carbonyl, nickel acetylacetonate or nickel acetate.

4. The process as claimed in any one of claims 1 to 3, wherein the promoter used is an alkali metal compound, organophosphonium compound or organoammonium compound in the form of its acetate or iodide.

5. The process as claimed in any one of claims 1 to 4, wherein the alkali metal compound used is a lithium salt.

6. The process as claimed in any one of claims 1 to 4, wherein the organophosphonium compound used is methyltributyl-, methyltriphenyl-, tetrabutyl- or dimethyldibutylphosphonium iodide.

7. The process as claimed in any one of claims 1 to 4, wherein the organoammonium compound used is N,N-dimethylimidazolium iodide, N-methylpyridinium iodide, N-methyl-3-picolinium iodide or N-methylquinolinium iodide.

8. The process as claimed in any one of claims 1 to 7, wherein the concentration of the non-noble metal from group VIII of the Periodic Table in the reaction mixture is set at between 0.01 and 0.75 mol/l, in particular at between 0.05 and 0.5 mol/l.

9. The process as claimed in any one of claims 1 to 8, wherein the concentration of the methyl iodide in the reaction mixture is set at between 0.1 and 7.5 mol/l, in particular at between 0.5 and 5 mol/l.

10. The process as claimed in any one of claims 1 to 9, wherein the concentration in the reaction mixture of the alkali metal compound, organophosphonium compound or organoammonium compound employed as promoter is set at between 0.05 and 4.5 mol/l, in particular at between 0.25 and 3.0 mol/l.

11. The process as claimed in any one of claims 1 to 10, wherein the copromoter used is a compound of Ti, Zr, V, Nb, Ta, Cr, Mo, W, Mn and Re.

12. The process as claimed in any one of claims 1 to 11, wherein the concentration of the copromoter in the reaction mixture is set at between 0.01 and 0.5 mol/l, in particular at between 0.05 and 0.3 mol.

13. The process as claimed in any one of claims 1 to 12, wherein up to 15 % by volume of hydrogen are added to the carbon monoxide employed.

14. The process as claimed in any one of claims 1 to 13, wherein the reaction is carried out under a pressure of from 50 to 100 bar and at a temperature of from 175 to 225°C.

15. The process as claimed in any one of claims 1 to 14, wherein the carbonylation process is carried out either continuously or batchwise.

## Revendications

1. Procédé de préparation simultanée d'acide acétique et d'anhydride acétique à partir d'un mélange de départ constitué de méthanol et d'acétate de méthyle ainsi que le cas échéant d'éther diméthylique par réaction avec du monoxyde de carbone dans une zone réactionnelle sur un système catalytique, dans des conditions anhydres, caractérisé en ce que :
a) on travaille en présence d'un système catalytique constitué d'un métal non noble du groupe VIII de la classification périodique, d'iodure de méthyle, d'un composé d'alcalin, organophosphonium ou organoammonium en tant que promoteur et le cas échéant d'un composé d'un métal non noble des groupes IV à VII de la classification périodique en tant que copromoteur;
b) on utilise en tant que mélange de départ un rapport molaire du méthanol à l'acétate de méthyle, ainsi que le cas échéant l'éther diméthylique, de 10 : 1 à 1 : 10 et
c) la réaction est effectuée sous une pression de 25 à 200 bars et à une température de 150 et 250°C.

2. Procédé selon la revendication 1, caractérisé en ce qu'on utilise le nickel en tant que métal non noble du groupe VIII de la classification périodique.

3. Procédé selon la revendication 1 ou 2, caractérisé en ce qu'on utilise en tant que composant catalytique l'iodure de nickel, le chlorure de nickel, le nickelcarbonyle, l'acétylacétonate de nickel ou l'acétate de nickel.

4. Procédé selon l'une des revendications 1 à 3, caractérisé en ce qu'on utilise en tant que promoteur un composé d'alcalin, organophosphonium ou organoammonium sous la forme de son acétate ou de son iodure.

5. Procédé selon l'une des revendications 1 à 4, caractérisé en ce qu'on utilise comme composé alcalin un sel de lithium.

6. Procédé selon l'une des revendications 1 à 4, caractérisé en ce qu'on utilise comme composé organophosphonium l'iodure de méthyltributylphosphonium, l'iodure de méthyltriphénylphosphonium, l'iodure de tétrabutylphosphonium ou l'iodure de diméthyldibutylphosphonium.

7. Procédé selon l'une des revendications 1 à 4, caractérisé en ce qu'on utilise comme composé organoammonium l'iodure de N,N-diméthylimidazolium, l'iodure de N-méthylpyridinium, l'iodure de N-méthyl-3-picolinium ou l'iodure de N-méthylquinoléinium.

8. Procédé selon l'une des revendications 1 à 7, caractérisé en ce qu'on fixe la concentration du métal non noble du groupe VIII de la classification périodique dans le mélange réactionnel à une valeur comprise entre 0,01 et 0,75, de préférence 0,05 et 0,5 mol/l.

9. Procédé selon l'une des revendications 1 à 8, caractérisé en ce qu'on fixe la concentration en iodure de méthyle dans le mélange réactionnel à une valeur comprise entre 0,1 et 7,5, de préférence 0,5 et 5 mol/l.

10. Procédé selon l'une des revendications 1 à 9, caractérisé en ce que la concentration du composé d'alcalin, organophosphonium ou organoammonium utilisé en tant que promoteur dans le mélange réactionnel est comprise entre 0,05 et 4,5, de préférence 0,25 et 3,0 mol/l.

11. Procédé selon l'une des revendications 1 à 10, caractérisé en ce qu'on utilise comme copromoteur des composés de Ti, Zr, V, Nb, Ta, Cr, Mo, W, Mn ou Re.

12. Procédé selon l'une des revendications 1 à 11, caractérisé en ce qu'on fixe la concentration du co-promoteur dans le mélange réactionnel à une valeur comprise entre 0,01 et 0,5, de préférence 0,05 et 0,3 mol/l.

13. Procédé selon l'une des revendications 1 à 12, caractérisé en ce qu'on ajoute au monoxyde de carbone utilise jusqu'à 15 % en volume d'hydrogène.

14. Procédé selon l'une des revendications 1 à 13, caractérisé en ce qu'on met en oeuvre la réaction sous une pression comprise entre 50 et 100 bars et à une température de 175 à 225°C.

15. Procédé selon l'une des revendications 1 à 14, caractérisé en ce que le procédé de carbonylation fonctionne aussi bien en continu qu'en discontinu.
